# EUROPEAN PATENT APPLICATION

(11) **EP 4 715 058 A1**
(43) Date of publication of application: **25.03.2026**
(21) Application number: 24806516.1
(22) Date of filing: 11.05.2024
(51) Int. Cl.: C12N 15/864, C12N 15/86, C12N 15/113, C07K 14/47, A61K 48/00, A61P 25/28

(54) **GENE THERAPY FOR RETT SYNDROME**

(30) Priority: 15.05.2023 CN 202310542967
(71) Applicant: Genecombio Ltd., Shanghai 200131 (CN); Chang, Bingsheng, Shanghai 200131 (CN)
(72) Inventor: JIAO, Guanyi, Shanghai 200131 (CN); CHANG, Bingsheng, Shanghai 200131 (CN)
(74) Representative: Ostertag & Partner Patentanwälte mbB
(86) International application number: PCT/CN2024/092672
(87) International publication number: WO 2024/235164

(57) **Abstract**

Provided are a gene expression cassette containing a polynucleotide sequence encoding a human MeCP2 protein, a viral vector carrying the gene expression cassette, and the use thereof in the treatment of Rett syndrome. One or more binding sites of miRNA are introduced into the provided gene expression cassette, so that overexpression of MeCP2 in brain tissue is prevented and the expression of MeCP2 in DRG and the liver is reduced, which solve the possible safety problems caused by the gene therapy.

## Description

### TECHNICAL FIELD

The present application relates to the field of gene therapy, and specifically to a gene expression cassette comprising a polynucleotide sequence encoding a human MeCP2 protein, a viral vector carrying the gene expression cassette, and use thereof in the treatment of Rett syndrome.

### BACKGROUND

Rett syndrome (RTT) is a severe neurological disorder with a prevalence of 1/15,000 to 1/10,000, caused by mutations or deletions in the X-linked Mecp2 gene. Since the mutated Mecp2 gene is mainly located on the paternal X chromosome, the number of female patients is far greater than that of male patients. The clinical features include progressive intellectual decline, autistic behaviors, stereotyped movements, and gait abnormalities, with obvious symptoms usually appearing 6 to 18 months after birth. Most patients with Rett syndrome can survive into adulthood but are unable to take care of themselves in daily life. Currently, there is no effective cure for RTT; only corresponding interventions can be conducted according to the different clinical stages of patients to alleviate symptoms, such as physical therapy, surgical treatment, antiepileptic treatment, etc.

Animal experiments have shown that reactivation of the Mecp2 gene can effectively reverse RTT-related symptoms in conditional Mecp2 gene-knockout mice. Therefore, introducing a normal Mecp2 gene via a vector to replace a defective Mecp2 gene may be a potential approach for treating Rett syndrome. However, low-level expression of the Mecp2 gene may fail to effectively rescue the symptoms, while overexpression of Mecp2 in brain tissue can cause toxicity. Thus, the challenge faced by this gene therapy method is how to transduce a sufficient number of neurons in brain tissue while avoiding harmful overexpression of Mecp2.

Over the past few decades, gene therapy based on adeno-associated virus (AAV) vectors has shown significant potential in the treatment of genetic diseases. Therefore, an exogenous Mecp2 gene can be introduced via an AAV vector to express normal MeCP2 protein in the body, thereby treating Rett syndrome at its root cause. However, previous studies have found that when AAV vectors enter the central nervous system of nonhuman primates (NHPs) through the blood or cerebrospinal fluid, they can induce toxicity in the dorsal root ganglion (DRG), with the most severe toxicity manifested as ataxia. Traditional immunosuppressive regimens cannot prevent this toxicity, possibly because it may be caused by high transduction efficiency, which in turn induces cellular stress due to excessive transgene products in target cells. Therefore, there is an urgent need to develop safer and more effective gene therapy drugs for the treatment of Rett syndrome.

It should be noted that the methods described in this section are not necessarily methods that have been previously conceived or employed. Unless otherwise specified, it shall not be assumed that any method described in this section is regarded as prior art merely by virtue of its inclusion in this section. Similarly, unless otherwise specified, the problems mentioned in this section should not be regarded as having been recognized in any prior art.

### SUMMARY

To address the above problems, the present application provides a gene expression cassette comprising a polynucleotide sequence encoding a human MeCP2 protein, wherein the gene expression cassette comprises at least one first miRNA binding site, and the first miRNA binding site includes one or more of a miR-22 binding site, a miR-132 binding site, a miR-124 binding site, a miR-483 binding site, or a miR-130 binding site. The gene expression cassette in the present application can express the correct MeCP2 protein in brain tissue by carrying the polynucleotide sequence encoding the human MeCP2 protein; meanwhile, by adding one or more first miRNA binding sites, the characteristic of miRNAs inhibiting gene expression at specific sites can be utilized to further regulate the expression level of a MeCP2 gene at the post-transcriptional level, thereby preventing overexpression of Mecp2 in brain tissue and avoiding toxicity. The gene expression cassette provided by the present application ensures a certain level of the MeCP2 protein expression in brain tissue while controlling the expression level of the MeCP2 protein in brain tissue to prevent it from being excessively high; and since either too low or too high expression levels of the MeCP2 protein in brain tissue cannot effectively treat Rett syndrome, the gene expression cassette provided by the present application has high technical difficulty and promising application prospects.

In some embodiments, the gene expression cassette further comprises a second miRNA binding site and/or a third miRNA binding site. The gene expression cassette in the present application reduces the expression of the MeCP2 protein in DRG and liver by adding one or more second miRNA binding sites and/or third miRNA binding sites, thereby alleviating DRG toxicity and liver toxicity caused by AAV vectors and reducing adverse reactions of the body to the AAV vectors and transgene products.

In some embodiments, the gene expression cassette further includes a promoter, and the promoter comprises at least one of a MeCP2 core promoter, a MeCP2 silencer element, or a CNS regulatory element. In some embodiments, the promoter includes MeP549, MeP426, MeP322, or MeP223. By selecting a specific promoter, the expression level of the MeCP2 protein in brain tissue can be further regulated to achieve a more appropriate expression level to meet the therapeutic needs for Rett syndrome.

In some embodiments, a length of the gene expression cassette does not exceed about 2.2 kb. The gene expression cassette in the present application has a relatively short length, thereby avoiding exceeding the carrying capacity limit of the AAV vectors due to the long gene length when the AAV vectors are used for delivery.

According to one embodiment of the present application, a recombinant vector is further provided, wherein the recombinant vector comprises the gene expression cassette described in the present application.

According to one embodiment of the present application, a pharmaceutical composition is further provided, wherein the pharmaceutical composition includes the gene expression cassette described in the present application and the recombinant vector described in the present application.

According to one embodiment of the present application, a use of the gene expression cassette described in the present application, the recombinant vector described in the present application, and the pharmaceutical composition described in the present application for expressing an MeCP2 protein in cells is further provided.

According to one embodiment of the present application, a use of the gene expression cassette described in the present application, the recombinant vector described in the present application, and the pharmaceutical composition described in the present application in the preparation of a drug for detecting, preventing, or treating a disease is further provided.

According to one embodiment of the present application, a method for detecting, preventing, or treating a disease is further provided, the method includes administering the gene expression cassette described in the present application, the recombinant vector described in the present application, or the pharmaceutical composition described in the present application to a subject in need thereof.

It should be understood that the content described in this section is not intended to identify key or important features of the embodiments of the present application, nor is it used to limit the scope of the present application. Other features of the present application will become easily understandable through the following specification.

### BRIEF DESCRIPTION OF THE DRAWINGS

The drawings exemplarily illustrate the embodiments and form part of the specification. Together with the textual description of the specification, they serve to explain the exemplary implementations of the embodiments. The shown embodiments are for illustrative purposes only and do not limit the scope of the claims. Throughout all the drawings, the same reference numerals refer to similar but not necessarily identical elements.

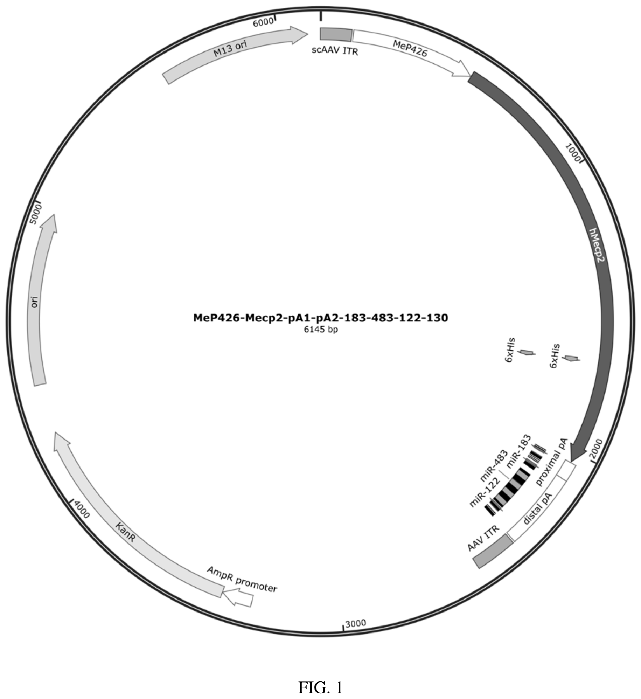

FIG. 1 shows a component diagram of the vector scAAV-MeP426-Mecp2-pA1-pA2-183-483-122-130 provided in Example 1 of the present application. The miRNA binding sites contained in the vector comprise: a miR-22 binding site located in pA1 (proximal termination sequence), a miR-132 binding site and miR-124 binding sites located in pA2 (distal termination sequence), and a miR-183 binding site, miR-483 binding site, miR-122 binding site, and miR-130 binding site located between pA1 and pA2.

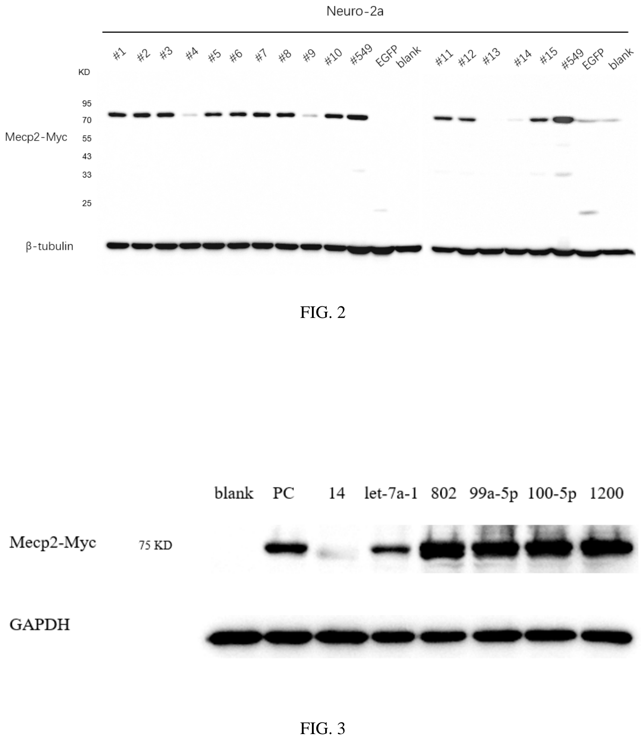

FIG. 2 shows a graph of the MeCP2 protein expression results after transfection of Neuro-2a cells with different vectors in Example 2.1. Among them, different labels in the figure represent transfection with different vectors: #1 represents scAAV-MeP223-Mecp2-pA1-pA2, #2 represents scAAV-MeP223-Mecp2-pA1-pA2-183-483-122, #3 represents scAAV-MeP223-Mecp2-pA1-pA2-183-483-122-1244, #4 represents scAAV-MeP223-Mecp2-pA1-pA2-183-483-122-130, #5 represents scAAV-MeP223-Mecp2-pA1-pA2-183-483-122-132, #6 represents scAAV-MeP322-Mecp2-pA1-pA2, #7 represents scAAV-MeP322-Mecp2-pA1-pA2-183-483-122, #8 represents scAAV-MeP322-Mecp2-pA1-pA2-183-483-122-124, #9 represents scAAV-MeP322-Mecp2-pA1-pA2-183-483-122-130, #10 represents scAAV-MeP322-Mecp2-pA1-pA2-183-483-122-132, #11 represents scAAV-MeP426-Mecp2-pA1-pA2, #12 represents scAAV-MeP426-Mecp2-pA1-pA2-183-483-122, #13 represents scAAV-MeP426-Mecp2-pA1-pA2-183-483-122-124, #14 represents scAAV-MeP426-Mecp2-pA1-pA2-183-483-122-130, #15 represents scAAV-MeP426-Mecp2-pA1-pA2-183-483-122-132, EGFP represents scAAV-MeP426-EGFP(c-myc)-pA49, #549 represents scAAV-MeP549-Mecp2-pA49, and "blank" represents an untransfected blank group.

FIG. 3 shows a graph of the MeCP2 protein expression results after transfection of Neuro-2a cells with different vectors in Example 2.2. Among them, different labels in the figure represent transfection with different vectors: "blank" represents the untransfected blank group, PC represents scAAV-MeP426-Mecp2-pA1-pA2, 14 represents scAAV-MeP426-Mecp2-pA1-pA2-183-483-122-130, Let-7a-1 represents scAAV-MeP426-Mecp2-pA1-pA2-let-7a-1, 802 represents scAAV-MeP426-Mecp2-pA1-pA2-802, 99a-5p represents scAAV-MeP426-Mecp2-pA1-pA2-99a-5p, 100-5p represents scAAV-MeP426-Mecp2-pA1-pA2-100-5p, and 1200 represents scAAV-MeP426-Mecp2-pA1-pA2-1200.

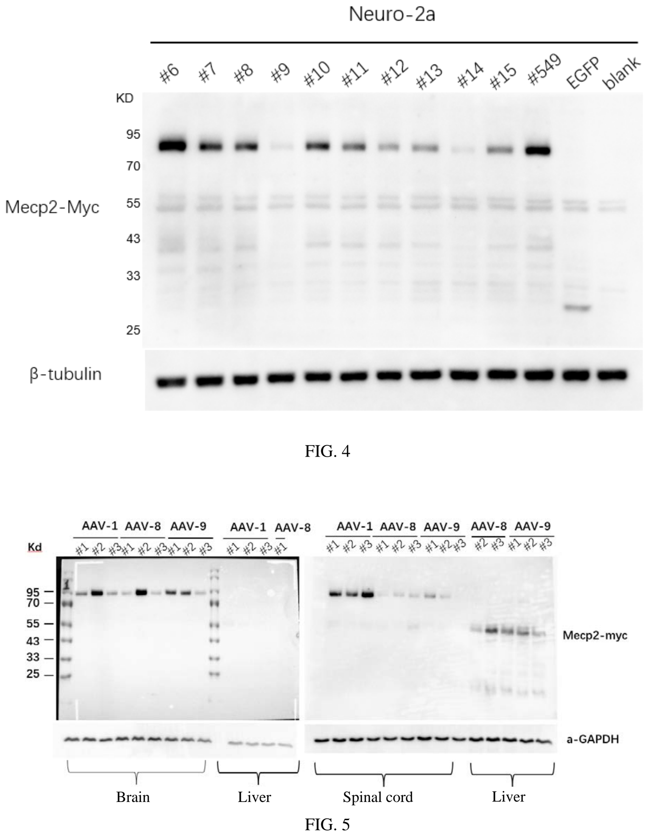

FIG. 4 shows a graph of the Mecp2 protein expression results after infection of Neuro-2a cells with different vectors packaged with a serotype AAV9 in Example 2.3. Among them, different labels in the figure represent infection with different vectors: #6 represents scAAV-MeP322-Mecp2-pA1-pA2, #7 represents scAAV-MeP322-Mecp2-pA1-pA2-183-483-122, #8 represents scAAV-MeP322-Mecp2-pA1-pA2-183-483-122-124, #9 represents scAAV-MeP322-Mecp2-pA1-pA2-183-483-122-130, #10 represents scAAV-MeP322-Mecp2-pA1-pA2-183-483-122-132, #11 represents scAAV-MeP426-Mecp2-pA1-pA2, #12 represents scAAV-MeP426-Mecp2-pA1-pA2-183-483-122, #13 represents scAAV-MeP426-Mecp2-pA1-pA2-183-483-122-124, #14 represents scAAV-MeP426-Mecp2-pA1-pA2-183-483-122-130, #15 represents scAAV-MeP426-Mecp2-pA1-pA2-183-483-122-132, EGFP represents scAAV-MeP426-EGFP(c-myc)-pA49, #549 represents scAAV-MeP549-Mecp2-pA49, and "blank" represents an untransfected blank group.
FIG. 5 shows a graph of the Mecp2 protein expression results in the brain, spinal cord, and liver of mice after infection with the vector scAAV-pMeP549-Mecp2-pA49 packaged with three serotypes (AAV1, AAV8, and AAV9) in Example 2.4.

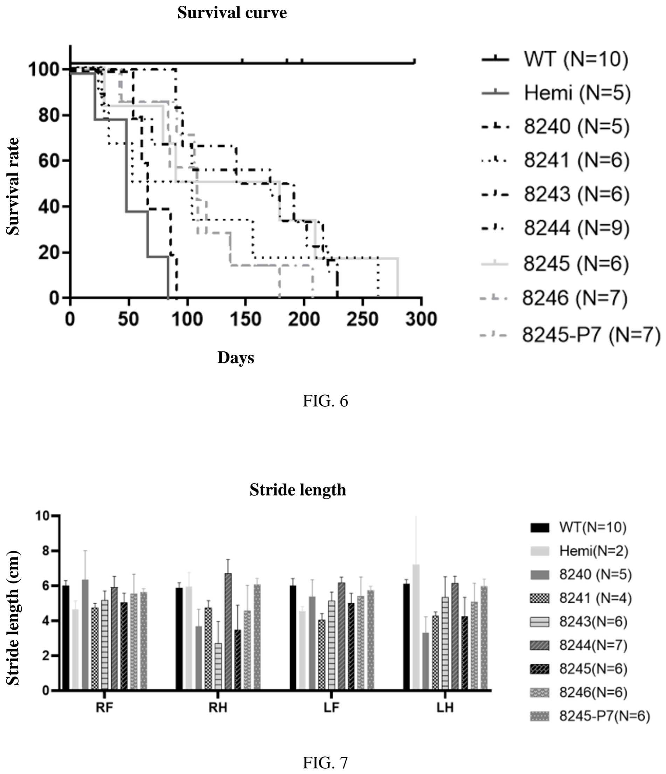

FIG. 6 shows a graph of the survival period results of Rett model mice in Example 3.3. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, a WT group represents wild-type mice without administration, a hemi group represents model mice without administration, and "vehicle" represents Rett model mice administered with a drug vehicle.
FIG. 7 shows a graph of the stride length results of Rett model mice in Example 3.4. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, a WT group represents wild-type mice without administration, a hemi group represents model mice without administration, and "vehicle" represents Rett model mice administered with a drug vehicle. In addition, RF represents the right forelimb of the mice, RH represents the right hindlimb, LF represents the left forelimb, and LH represents the left hindlimb.

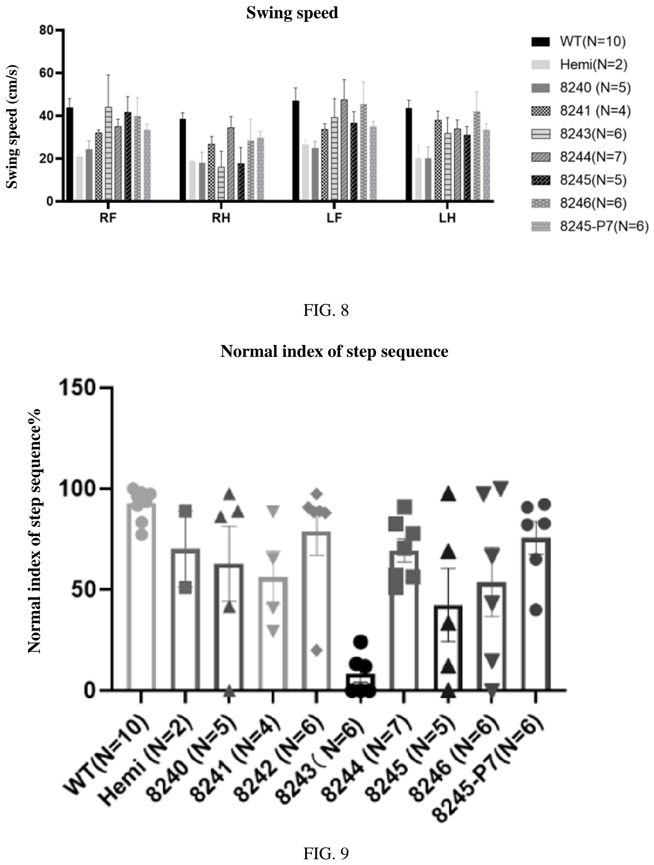

FIG. 8 shows a graph of the swing speed results of Rett model mice in Example 3.4. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, a WT group represents wild-type mice without administration, a hemi group represents model mice without administration, and "vehicle" represents Rett model mice administered with a drug vehicle. In addition, RF represents the right forelimb of the mice, RH represents the right hindlimb, LF represents the left forelimb, and LH represents the left hindlimb.
FIG. 9 shows a graph of the regularity index (RI) results of Rett model mice in Example 3.4. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, a WT group represents wild-type mice without administration, a hemi group represents model mice without administration, and "vehicle" represents Rett model mice administered with a drug vehicle. In addition, RF represents the right forelimb of the mice, RH represents the right hindlimb, LF represents the left forelimb, and LH represents the left hindlimb.

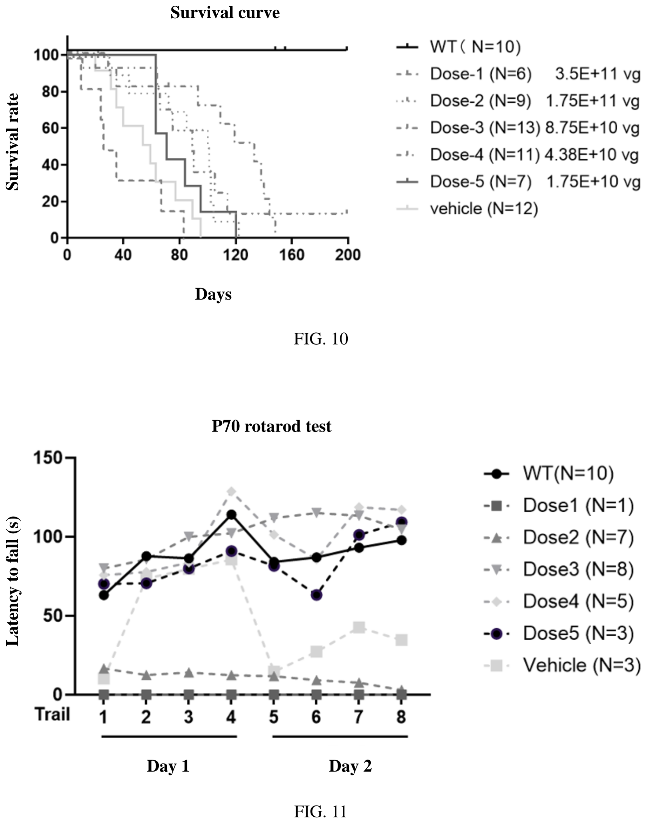

FIG. 10 shows a graph of the survival period results of Rett model mice in Example 4.1. Among them, different labels in the figure represent treatment with different doses of vector 8244: Dose 1 represents 3.5×10¹¹ vg/mouse, Dose 2 represents 1.75x10¹¹ vg/mouse, Dose 3 represents 8.75×10¹⁰ vg/mouse, Dose 4 represents 4.38×10¹⁰ vg/mouse, Dose 5 represents 1.75×10¹⁰ vg/mouse, WT represents wild-type mice without administration, and "vehicle" represents Rett model mice administered with a drug vehicle.
FIG. 11 shows a graph of the rotarod test results of Rett model mice in Example 4.2. Among them, different labels in the figure represent treatment with different doses of vector 8244: Dose 1 represents 3.5×10¹¹ vg/mouse, Dose 2 represents 1.75x10¹¹ vg/mouse, Dose 3 represents 8.75×10¹⁰ vg/mouse, Dose 4 represents 4.38×10¹⁰ vg/mouse, Dose 5 represents 1.75×10¹⁰ vg/mouse, WT represents wild-type mice without administration, and "vehicle" represents Rett model mice administered with a drug vehicle.

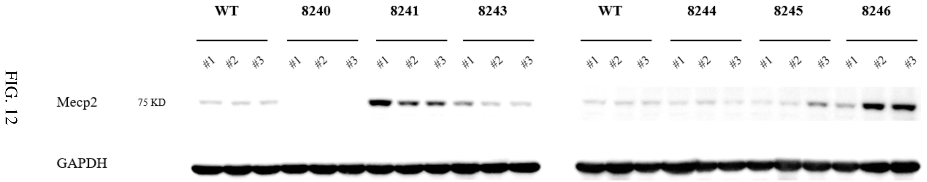

FIG. 12 shows a graph of the Mecp2 protein expression results in the brain and spinal cord of mice in Example 5.1. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, WT represents wild-type mice without administration, and "vehicle" represents Rett model mice administered with a drug vehicle.

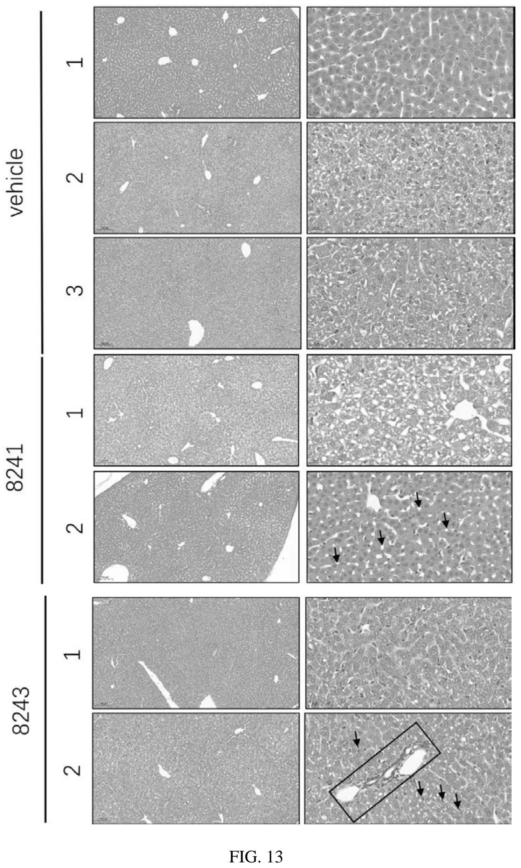

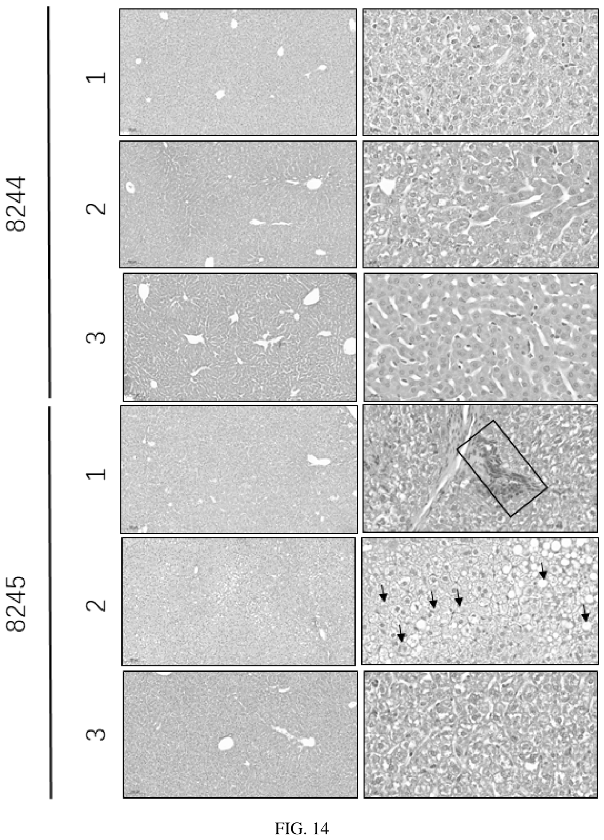

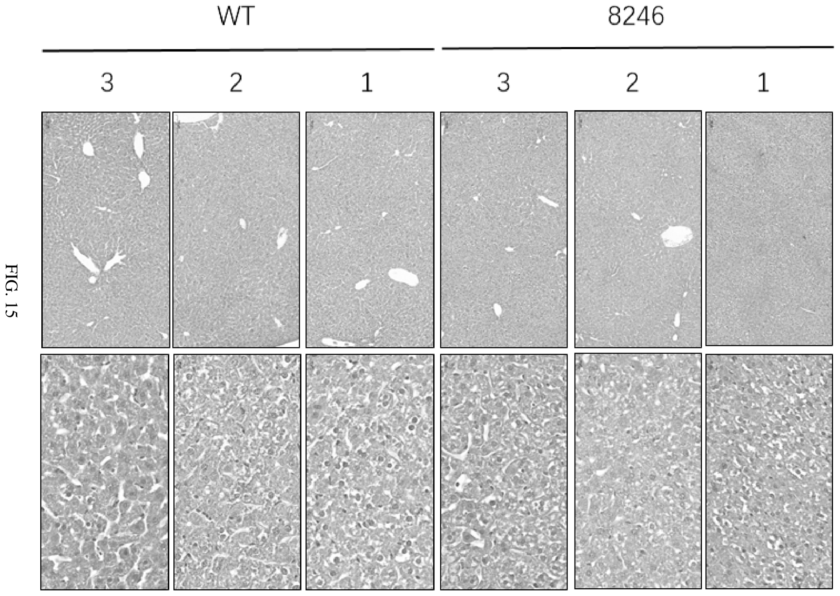

FIGS. 13, 14, and 15 show a graph of the pathological section staining results of mouse liver tissue in Example 5.2. Among them, different labels in the figure represent treatment with different vectors: 8241 represents scAAV9-MeP549-Mecp2-pA49, 8243 represents scAAV9-MeP322-Mecp2-pA1-pA2-183-483-122-130, 8244 represents scAAV9-MeP426-Mecp2-pA1-pA2-183-483-122-130, 8245 represents scAAV9-MeP426-Mecp2-pA1-pA2-183-483-122-132, 8246 represents scAAV1-MeP426-Mecp2-pA1-pA2-183-483-122-132, WT represents wild-type mice without administration, and "vehicle" represents Rett model mice administered with a drug vehicle.

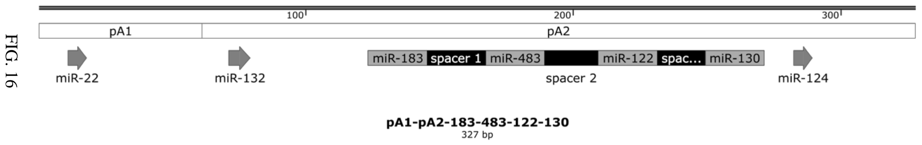

FIG. 16 shows a schematic diagram of miRNA binding sites in the vector scAAV-MeP426-Mecp2-pA1-pA2-183-483-122-130 provided in Example 1 of the present application. Among them, miR-22 represents a miR-22 binding site, miR-132 represents a miR-132 binding site, miR-124 represents a miR-124 binding site, miR-183 represents a miR-183 binding site, miR-483 represents a miR-483 binding site, miR-122 represents a miR-122 binding site, miR-130 represents a miR-130 binding site, and "spacer" represents a spacer sequence.

### DETAILED DESCRIPTION OF EMBODIMENTS

Unless otherwise indicated, all numbers representing content, concentration, ratio, weight, particle diameter, percentage, technical effect, and so forth as used in the specification and claims are to be understood as being modified in any case by the term "about" or "approximately". Accordingly, unless indicated to the contrary, numerical parameters as set forth in the following specification and appended claims are approximations. Unless otherwise indicated, terms as used herein have the meanings commonly understood by those skilled in the art. For those skilled in the art, each numerical parameter may vary depending upon the desired properties and effects sought to be obtained by the present disclosure and should be construed in accordance with the number of significant figures and ordinary rounding techniques or in a manner understood by those skilled in the art.

Although the broad range of the numerical values and the parameters described in the present disclosure are approximate, the numerical values described in the specific embodiments are provided as accurately as possible. However, any numerical value inherently contains certain errors, which are inevitably caused by the standard deviation found in their respective test measurements. Each numerical range given in the specification will include every narrower numerical range that falls within such broader numerical range, as if such narrower numerical ranges were all explicitly written herein.

Unless otherwise indicated or contradictory to the context, the terms or expressions used herein should be read in conjunction with the entire content of the present disclosure and as understood by those skilled in the art. All technical and scientific terms used herein have the same meanings as commonly understood by those skilled in the art, unless otherwise defined.

As used herein, the expression "A and/or B" includes three cases: (1) A; (2) B; and (3) A and B. The expression "A, B and/or C" includes seven cases: (1) A; (2) B; (3) C; (4) A and B; (5) A and C; (6) B and C; and (7) A, B and C. The meaning of similar expressions may be deduced by analogy.

As used herein, "nucleic acid" and "polynucleotide" are used interchangeably and refer to a polymeric form of nucleotides of any length, including deoxyribonucleotides, ribonucleotides, combinations thereof, and analogs thereof.

As used herein, "polypeptide" and "peptide" are used interchangeably and refer to a polymer of amino acids of any length. Thus, polypeptides, oligopeptides, proteins, antibodies, and enzymes are all included within the definition of polypeptides.

It should be noted that in the context of the present application, "upstream" refers to the 5'-end of a gene or the N-terminus of a protein, and "downstream" refers to the 3'-end of a gene or the C-terminus of a protein. The direction from upstream to downstream is from the 5'-end to the 3'-end or from the N-terminus to the C-terminus.

As used herein, a "vector" refers to a nucleic acid molecule capable of transporting another nucleic acid molecule to which it is linked. Examples of vectors include, but are not limited to, plasmids, viruses, bacteria, bacteriophages, and insertable DNA fragments.

As used herein, an "adeno-associated viral vector" is derived from an adeno-associated virus. An "adeno-associated viral vector" is derived from the wild-type genome of the virus by using molecular methods to remove all or part of the wild-type genome and replace it with heterologous (non-native) nucleic acid (e.g., a nucleic acid encoding a therapeutic protein or polynucleotide sequence). Typically, for adeno-associated viral vectors, one or both inverted terminal repeats (ITRs) from the adeno-associated viral genome are retained in the adeno-associated viral vector. Adeno-associated viral vectors can be used as gene therapy vectors because they can introduce nucleic acid/genetic material into cells, allowing the nucleic acid/genetic material to remain in the cells.

As used herein, a "serotype" refers to an adeno-associated virus with a capsid that is serologically distinct from other adeno-associated viral serotypes. Serological specificity is determined based on the lack of cross-reactivity between antibodies against one adeno-associated virus compared with another adeno-associated virus. Such differences in cross-reactivity are typically caused by differences in capsid protein sequences/antigenic determinants (e.g., due to differences in the VP1, VP2, and/or VP3 sequences of the adeno-associated viral serotypes). Under the traditional definition, a serotype means that the virus of interest has been tested for neutralizing activity against sera specific for all existing and characterized serotypes, and no antibodies that neutralize the virus of interest have been found. As more naturally occurring viral isolates are discovered and/or capsid mutants are generated, there may or may not be serological differences from any of the currently existing serotypes. Therefore, in cases where a new virus (e.g., an adeno-associated virus) does not have serological differences, such a new virus (e.g., an adeno-associated virus) will be a subgroup or variant of the corresponding serotype. In many cases, serological testing for neutralizing activity has not been performed on mutant viruses with modified capsid sequences to determine whether they are another serotype under the traditional serotype definition. Therefore, for convenience and to avoid redundancy, the term "serotype" is used broadly to refer to serologically distinct viruses (e.g., adeno-associated viruses) as well as viruses (e.g., adeno-associated viruses) that may be within a subgroup or variant of a given serotype and are not serologically distinct.

As used herein, "miRNA (microRNA)" refers to a single-stranded non-coding RNA of a certain length. RNA-induced silencing complex formed by miRNAs and protein factors can recognize target sequences in mRNA and reduce the expression level of mRNA by degrading mRNA molecules, promoting 3'-end deadenylation of mRNA molecules, and inhibiting translation, thereby regulating gene expression at the post-transcriptional level (Kim VN. Nat Rev Mol Cell Biol. 2005; 6(5): 376-385.).

As used herein, "miRNA target sequence" and "miRNA binding site" are used interchangeably and refer to a nucleotide sequence that can bind to a specific miRNA. In some non-limiting embodiments of the present application, the miRNA target sequence is designed in the 3'-terminal transcription termination sequence of an Mecp2 gene, which can bind to a specific miRNA to inhibit the expression level of the Mecp2 gene.

As used herein, "operably linked" refers to the linkage of multiple nucleic acid fragments in a functional relationship. A nucleic acid is "operably linked" to another nucleic acid sequence when it forms a functional relationship with the other nucleic acid sequence. For example, if a promoter or other transcriptional regulatory sequence affects the transcription of a coding sequence (or gene), it can be operationally linked to the coding sequence in a way that induces transcription of the gene. "operably linked" means that the linked nucleotide sequences may be either contiguous or non-contiguous.

The terms "alleviation" and "treatment" and their synonyms as used herein refer to the amelioration of a disease, disorder and/or condition. "Alleviation" and "treatment" may be an improvement in at least one measurable physical parameter that is not necessarily identifiable by the patient. "Alleviation" and "treatment" may also be the physical (e.g., stabilization of recognizable symptoms), physiological (e.g., stabilization of physical parameters), or both inhibition of the development of the disease, disorder, and/or condition. "Alleviation" and "treatment" may also refer to slowing the progression of or reversal of a disease, disorder and/or condition.

As used herein, the term "prevention" and its synonyms refer to delaying the onset of specific diseases, disorders, and/or conditions or the symptoms associated with these diseases, disorders, and/or conditions, or reducing the risk of acquiring these diseases, disorders, and/or conditions.

To make the above objects, features, and advantages of the present application more clearly understandable, the specific embodiments of the present application are described in detail below.

### Gene Expression Cassette

According to one embodiment of the present application, provided is a gene expr ession cassette comprising a polynucleotide sequence encoding a human MeCP2 protei n, wherein the gene expression cassette comprises at least one first miRNA binding si te. In some non-limiting embodiments, the human MeCP2 protein comprises the amin o acid sequence as shown in SEQ ID NO: 1. In some non-limiting embodiments, the MeCP2 protein may additionally comprise (e.g., at the C-terminus of the molecule) a heterologous (i.e., non-MeCP2) sequence, such as an epitope tag C-myc that facilitates isolation or identification. The sequence of the MeCP2 protein after adding C-myc is as follows (the underlined part is the heterologous sequence that is non-MeCP2): M AAAAAAAPSGGGGGGEEERLEEKSEDQDLQGLKDKPLKFKKVKKDKKEEKEGKHE PVQPSAHHSAEPAEAGKAETSEGSGSAPAVPEASASPKQRRSIIRDRGPMYDDPTLPE GWTRKLKQRKSGRSAGKYDVYLINPQGKAFRSKVELIAYFEKVGDTSLDPNDFDFT VTGRGSPSRREQKPPKKPKSPKAPGTGRGRGRPKGSGTTRPKAATSEGVQVKRVLEK SPGKLLVKMPFQTSPGGKAEGGGATTSTQVMVIKRPGRKRKAEADPQAIPKKRGRKP GSVVAAAAAEAKKKAVKESSIRSVQETVLPIKKRKTRETVSIEVKEVVKPLLVSTLGE KSGKGLKTCKSPGRKSKESSPKGRSSSASSPPKKEHHHHHHHSESPKAPVPLLPPLPPP PPEPESSEDPTSPPEPQDLSSSVCKEEKMPRGGSLESDGCPKEPAKTQPAVATAATAAE KYKHRGEGERKDIVSSSMPRPNREEPVDSRTPVTERVSGSSGSSGEQKLISEEDL_{∘}

In some embodiments, the first miRNA binding site includes at least one or more of a miR-22 binding site, a miR-132 binding site, a miR-124 binding site, a miR-483 binding site, or a miR-130 binding site. In some preferred embodiments, the first miRNA binding site includes at least one of the following groups: (1) a miR-22 binding site, a miR-132 binding site, a miR-124 binding site, and a miR-483 binding site; and/or (2) a miR-22 binding site, a miR-132 binding site, a miR-124 binding site, a miR-483 binding site, and a miR-130 binding site. In some more preferred embodiments, the first miRNA binding site includes at least one of the following groups: (1) 1 miR-22 binding site, 1 miR-132 binding site, 1 miR-124 binding site, and 1 miR-483 binding site; (2) 1 miR-22 binding site, 1 miR-132 binding site, 2 miR-124 binding sites, and 1 miR-483 binding site; (3) 1 miR-22 binding site, 1 miR-132 binding site, 1 miR-124 binding site, 1 miR-483 binding site, and 1 miR-130 binding site; and/or (4) 1 miR-22 binding site, 2 miR-132 binding sites, 1 miR-124 binding site, and 1 miR-483 binding site. In some embodiments, the first miRNA binding site can reduce the expression efficiency of the polynucleotide sequence encoding the human MeCP2 protein in human brain tissue. The presence of multiple miRNA binding sites can lead to the cooperative effect of multiple RISCs and provide efficient expression inhibition.

The 3'-transcription termination sequences in brain tissue mainly have two lengths: about 200 bp and about 8.5 kb, and a content of mRNA with a length of about 8.5 kb exceeds 80%. To better exert the functions of the two types of transcription termination signals, the two transcription termination sequences are combined. In some embodiments, the first miRNA binding site is located in a 3' transcription termination sequence of the gene expression cassette, and the 3' transcription termination sequence includes a distal termination sequence and/or a proximal termination sequence. In some embodiments, the proximal termination sequence (as shown in SEQ ID NO: 2) comprises a miR-22 binding site. In some non-limiting embodiments, the following is the proximal termination sequence, where the underlined part is the miRNA binding site: acaagaataaaggcagctgttgtctcttctccttatgggtagggctctgacaaagcttccc. In some embodiments, the distal termination sequence (as shown in SEQ ID NO: 3) comprises at least one of a miR-132 binding site, a miR-124 binding site, a miR-483 binding site, or a miR-130 binding site.

In some embodiments, the distal termination sequence further includes an upstrea m sequence element and a core upstream element. In the distal termination sequence, a length between the upstream sequence element (USE) and the core upstream elemen t (CSE) is maintained at 5-50 bp, and multiple miRNA binding sites are integrated th erein to regulate the expression of MeCP2. In some non-limiting embodiments, the fol lowing is the distal termination sequence, where the underlined part is the upstream s equence element and the core upstream element: atatcaccaggactgttaatatatttaaaaacaaaaggc aatttattaaggaaatttgtaccatttcagtaaacctgtctgaatgtacctgtatacgtttcaaaaacaccccccccccactgaatccctgta acctatttattatataaagagtttgccttataaatttacataaaaatgtccgtttgtgtcttttgttg. In some embodiments, the miR-483 binding site (as shown in SEQ ID NO: 4) and the miR-130 binding site (a s shown in SEQ ID NO: 5) are located between the upstream sequence element and the core upstream element. In some embodiments, the miR-132 binding site (as shown in SEQ ID NO: 6) and the miR-124 binding site (as shown in SEQ ID NO: 7) are not located between the upstream sequence element and the core upstream element.

In some embodiments, at least one thymine nucleotide in the miR-483 and miR-130 binding sites is replaced with an adenine nucleotide. In some non-limiting embodiments, to enhance the inhibitory effect of the miRNA binding sites, according to the functional characteristics of an Ago2 protein, the nucleotide at the first position of the 3'-end of the miR-483 and miR-130 binding sites is replaced with an adenine nucleotide. In some non-limiting embodiments, in the following sequence, the underlined part indicates the nucleotide position where miR-483 is replaced with an adenine nucleotide: CTCCCTTCTTTCCTCCCGTCT**a.** In some non-limiting embodiments, in the following sequence, the underlined part indicates the nucleotide position where miR-130 is replaced with an adenine nucleotide: ATGCCCTTTTAACATTGCACT**a**. In some preferred embodiments, the nucleotide sequence of the miR-22 binding site includes GGCAGCT; the miR-132 binding site includes the nucleotide sequence shown in SEQ ID NO: 6; the miR-124 binding site includes the nucleotide sequence shown in SEQ ID NO: 7; the miR-483 binding site includes the nucleotide sequence shown in SEQ ID NO: 4; and/or the miR-130 binding site includes the nucleotide sequence shown in SEQ ID NO: 5.

In some embodiments, the gene expression cassette further comprises a second miRNA binding site and/or a third miRNA binding site. In some embodiments, the second miRNA binding site can reduce the expression efficiency of the polynucleotide sequence encoding the human MeCP2 protein in human dorsal root ganglion, and the third miRNA binding site can reduce the expression efficiency of the polynucleotide sequence encoding the human MeCP2 protein in human liver tissue. The presence of multiple miRNA binding sites can lead to the cooperative effect of multiple RISCs and provide efficient expression inhibition. In some embodiments, the second miRNA binding site and/or the third miRNA binding site includes one or more of a miR-183 binding site, a miR-182 binding site, a miR-96 binding site, a miR23b binding site, a miR-145 binding site, a miR-148a binding site, a miR-22 binding site, a miR-122 binding site, a miR-143 binding site, a miR-21 binding site, or a miR-192 binding site. In some preferred embodiments, the second miRNA binding site includes a miR-183 binding site. In some preferred embodiments, the third miRNA binding site includes a miR-122 binding site. In some preferred embodiments, the miR-183 binding site includes the nucleotide sequence shown in SEQ ID NO: 9. In some preferred embodiments, the miR-122 binding site includes the nucleotide sequence shown in SEQ ID NO: 10.

In some embodiments, the first miRNA binding site, the second miRNA binding site, and/or the third miRNA binding site are operably linked via a spacer sequence. In some embodiments, the spacer sequence comprises the nucleotide sequence shown in any one of SEQ ID NOs: 11-13. In some non-limiting embodiments, endogenous sequences of 22 bp, 20 bp, and 18 bp are selected as spacer sequences to enhance the cooperative effect of different miRNAs.

In the gene expression cassette of the present application, the first miRNA binding site, the second miRNA binding site, and the third miRNA binding site can be combined in any suitable manner. In some preferred embodiments, the gene expression cassette includes at least one of the following groups: (1) a miR-22 binding site, a miR-132 binding site, a miR-124 binding site, a miR-483 binding site, a miR-183 binding site, and a miR-122 binding site; and/or (2) a miR-22 binding site, a miR-132 binding site, a miR-124 binding site, a miR-483 binding site, a miR-130 binding site, a miR-183 binding site, and a miR-122 binding site. In some more preferred embodiments, the gene expression cassette includes at least one of the following groups: (1) 1 miR-22 binding site, 1 miR-132 binding site, 1 miR-124 binding site, 1 miR-483 binding site, 1 miR-183 binding site, and 1 miR-122 binding site; (2) 1 miR-22 binding site, 1 miR-132 binding site, 2 miR-124 binding sites, 1 miR-483 binding site, 1 miR-183 binding site, and 1 miR-122 binding site; (3) 1 miR-22 binding site, 1 miR-132 binding site, 1 miR-124 binding site, 1 miR-483 binding site, 1 miR-130 binding site, 1 miR-183 binding site, and 1 miR-122 binding site; and/or (4) 1 miR-22 binding site, 2 miR-132 binding sites, 1 miR-124 binding site, 1 miR-483 binding site, 1 miR-183 binding site, and 1 miR-122 binding site.

In some embodiments, the gene expression cassette further includes a promoter. The promoter can be any suitable promoter sequence, i.e., a nucleic acid sequence recognizable by the host cell for expressing the nucleic acid sequence. The promoter sequence contains transcriptional regulatory sequences that mediate the expression of proteins or polypeptides. The promoter can be any nucleic acid sequence with transcriptional activity in the selected host cell, including mutated, truncated, and hybrid promoters, which can be derived from genes encoding extracellular or intracellular proteins or polypeptides that are homologous or heterologous to the host cell. In some non-limiting embodiments, the host cell is a neuron , and the promoter can be a nucleic acid sequence with transcriptional activity in neuron. In some embodiments, the promoter comprises at least one of a MeCP2 core promoter, a MeCP2 silencer element, or a CNS regulatory element. In some preferred embodiments, the promoter is selected from at least one of MeP549 (as shown in SEQ ID NO: 14), MeP426 (as shown in SEQ ID NO: 15), MeP322 (as shown in SEQ ID NO: 16), and MeP223 (as shown in SEQ ID NO: 17).

In some embodiments, the 3'-transcription termination sequence further comprises an AU-rich element. The AU-rich element can be used to regulate the stability of mRNA. In some embodiments, the 3'-transcription termination sequence further comprises a termination signal. Any transcription termination sequence known in the art can be used in the present application. In some non-limiting embodiments, the termination signal is a synthetic termination signal (as shown in SEQ ID NO: 8).

In some embodiments, a length of the gene expression cassette does not exceed about 2.2 kb. The length of the gene expression cassette does not exceed the maximum carrying capacity of an AAV vector, facilitating delivery using various vectors including an AAV vector.

### Recombinant Vectors, Pharmaceutical Compositions

According to one embodiment of the present application, a recombinant vector is further provided, wherein the recombinant vector comprises the gene expression cassette described in the present application. Any suitable vector may be used for delivering the gene expression cassette. In some embodiments, the recombinant vector includes a recombinant plasmid expression vector or a recombinant viral vector. In some embodiments, the recombinant viral vector includes a recombinant adenoviral vector, a recombinant adeno-associated viral vector, a recombinant retroviral vector, a recombinant herpes simplex viral vector, or a recombinant vaccinia viral vector. In some embodiments, the recombinant viral vector is a recombinant adeno-associated viral vector. The recombinant adeno-associated viral vector may be of any suitable serotype. In some embodiments, the serotype of the recombinant adeno-associated viral vector includes AAV1, AAV2, AAV3, AAV4, AAV5, AAV6, AAV7, AAV8, AAV9, AAVrh10, AAV11, AAV12, AAV13, AAV-PHP.B, AAV-PHP.EB, AAVhu37, AAVrh74, or AAVhu68. In some embodiments, the serotype of the recombinant adeno-associated viral vector includes AAV1, AAV8, or AAV9. In some non-limiting embodiments, the recombinant viral vector is a serotype targeting neuron. The nucleotide sequences of the genomes of AAV serotypes are known in the art. In some embodiments, the gene expression cassette is located downstream of the 5' inverted terminal repeat sequence of the adeno-associated viral vector.

Viral particles comprising the gene expression cassette described in the present application are typically produced in packaging cells, which are capable of replicating the viral genome, expressing viral proteins, and assembling viral particles. Techniques for producing AAV vector particles in packaging cells are well-known in the art. In some non-limiting embodiments, packaging cells can be produced by simply transfecting suitable cells with one or more plasmids encoding the AAV genome, AAV proteins, and any required helper virus functions, known as the so-called "triple transfection" method: three plasmids are used, each carrying a set of such genes. See Grieger et al., Nature Protocols, 2006, 1(3): 1412-28.

According to one embodiment of the present application, a pharmaceutical composition is further provided, wherein the pharmaceutical composition includes the gene expression cassette described in the present application and the recombinant vector described in the present application. In some embodiments, the pharmaceutical composition is in the form of a lateral intraventricular injection drug, an intravenous injection drug, or an intrathecal injection drug. In addition to the gene expression cassette and/or recombinant vector provided herein, the pharmaceutical composition of the present application may further comprise a pharmaceutically, nutritionally, or physiologically acceptable carrier, such as a liquid, gel, or solid carrier, an aqueous vehicle, a non-aqueous vehicle, an antimicrobial agent, an isotonic agent, a buffer, an antioxidant, a suspending agent, a dispersing agent, a chelating agent, a diluent, an adjuvant, an excipient, or a non-toxic auxiliary substance, as well as other components known in the art or various combinations thereof.

### Uses

According to one embodiment of the present application, a use of the gene expression cassette described in the present application, the recombinant vector described in the present application, and the pharmaceutical composition described in the present application for expressing an MeCP2 protein in cells is further provided. The cells may be any cells of interest. In some embodiments, the cells are animal cells. In some embodiments, the animal cells include Neuro-2a cells.

According to one embodiment of the present application, a use of the gene expression cassette described in the present application, the recombinant vector described in the present application, and the pharmaceutical composition described in the present application in the preparation of a drug for detecting, preventing, or treating a disease is further provided. In some embodiments, the disease is an MeCP2-related disease. In some embodiments, the MeCP2-related disease includes: a disease related to insufficient MeCP2 protein expression, a disease related to MeCP2 protein deficiency, a disease related to MeCP2 gene deletion, or a disease related to MeCP2 gene mutation. In some embodiments, the MeCP2-related disease is Rett syndrome. In some embodiments, the drug includes an intraventricular injection drug, an intravenous injection drug, or an intrathecal injection drug.

### Disease Treatment Methods

According to one embodiment of the present application, a method for detecting, preventing, or treating a disease is further provided, the method includes administering the gene expression cassette described in the present application, the recombinant vector described in the present application, or the pharmaceutical composition described in the present application to a subject in need thereof. In some embodiments, the disease is an MeCP2-related disease. In some embodiments, the MeCP2-related disease includes: a disease related to insufficient MeCP2 protein expression, a disease related to MeCP2 protein deficiency, a disease related to MeCP2 gene deletion, or a disease related to MeCP2 gene mutation. In some embodiments, the MeCP2-related disease is Rett syndrome.

The various embodiments and preferred options of the present application described above may be combined with each other (provided that they are not inherently contradictory to each other) and are applicable to the uses of the present application. All various embodiments formed by such combinations shall be deemed as part of the present application.

### Examples

The following provides an explanation of exemplary embodiments of the present application in conjunction with the accompanying drawings, including various details of the embodiments of the present application to aid understanding. It should be understood that these embodiments are merely exemplary and are in no way intended to limit the protection scope of the present application. The protection scope of the present application is only defined by the claims. Therefore, those skilled in the art should recognize that various changes and modifications can be made to the embodiments described herein without departing from the scope of the present application. Similarly, for clarity and conciseness, descriptions of well-known functions and structures are omitted in the following description.

Unless otherwise indicated, the reagents and instruments used in the following embodiments are all conventional products that are commercially available. Unless otherwise indicated, experiments are performed under conventional conditions or conditions recommended by the manufacturer.

### Example 1: Construction of Expression Vectors

### 1.1 Construction of Plasmid Vectors

Tsingke Biotech was commissioned to synthesize relevant elements, and all these elements were constructed into a cloning vector resistant to puc57 ampicillin. The synthesis steps of the relevant elements were as follows:
A CDS sequence of an Mecp2 gene encoding an MeCP2 protein (the amino acid sequence is shown in SEQ ID NO: 1) was combined with the endogenous 3' UTR to generate Mecp2-pA1-pA2 (the nucleotide sequence was shown in SEQ ID NO: 18). Among them: the endogenous 3' UTR comprised a proximal termination sequence (pA1) and a distal termination sequence (pA2); pA1 comprised a miR-22 binding site, and pA2 comprised miR-132 and miR-124 binding sites. The MeCP2 protein may comprise a C-myc tag at the C-terminus of the molecule for subsequent identification of expression efficiency. 4 µg of the synthesized plasmid was diluted with 40 µL of ddH₂O to a concentration of 100 ng/µL for subsequent PCR experiments.

### 1.2 Construction of scAAV Vectors

The Mecp2-pA1-pA2 constructed in 1.1, together with neuron -specific promoters MeP549 (sequence shown in SEQ ID NO: 14), MeP426 (sequence shown in SEQ ID NO: 15), MeP322 (sequence shown in SEQ ID NO: 16), and MeP223 (sequence shown in SEQ ID NO: 17), were used to construct the following plasmid vectors: scAAV-MeP426-Mecp2-pA1-pA2, scAAV-MeP322-Mecp2-pA1-pA2, and scAAV-MeP223-Mecp2-pA1-pA2.

Meanwhile, a 49 bp termination sequence (pA49) without an miRNA binding site was used to synthesize scAAV-MeP549-Mecp2-pA49 as a control.

The detailed construction steps were as follows:
(1) An scAAV backbone plasmid (PackGene Biotech, EC021) was digested with ScaI-HF (NEB, R3122V).
(2) A fast and high-fidelity premixed PCR enzyme KOD One PCR Master Mix (TOYOBO, KMM-201) was used for cloning experiments. An 18-20 bp microhomologous sequence was introduced between different fragments or between a fragment and a vector via PCR for subsequent seamless cloning experiments.
(3) Construction of scAAV-MeP549-Mecp2-pA49: MeP549, Mecp2, and pA49 were amplified by PCR, and their templates and primers were shown in Table 1. The above amplification products were ligated to a linear scAAV backbone by seamless cloning (Clone Smarter, C5891-50) to construct an scAAV-MeP549-Mecp2-pA49 plasmid (the sequence of the exogenous nucleic acid fragment between its ITR sequences was shown in SEQ ID NO: 20). The ligation product was transformed into recombinase-deficient E. coli competent cells stabl3 (TransGen, CD521-01) for amplification.

**Table 1: PCR primer sequences for scAAV-MeP549-Mecp2-pA49**

| Name | Template sequence | Primer sequence |
|---|---|---|
| MeP549 | MeP549 (SEQ ID NO: 14) | MeP549-F (SEQ ID NO: 22) |
| | | MeP549-R (SEQ ID NO: 23) |
| Mecp2 | Mecp2-pA1-pA2 (SEQ ID NO: 18) | Mecp2-F (SEQ ID NO: 24) |
| | | Mecp2-R (SEQ ID NO: 25) |
| pA49 | Amplified using the two pA49 primers as templates | pA49-F (SEQ ID NO: 26) |
| | | pA49-R (SEQ ID NO: 27) |

(4) Construction of scAAV-MeP426-Mecp2-pA1-pA2, scAAV-MeP322-Mecp2-pA1-pA2, and scAAV-MeP223-Mecp2-pA1-pA2: MeP426, MeP322, MeP226, and Mecp2-pA1-pA2 were amplified by PCR, and their templates and primers were shown in Table 2. The above amplification products were ligated to the linear scAAV backbone via seamless cloning (Clone Smarter, C5891-50) to construct scAAV-MeP426-Mecp2-pA1-pA2, scAAV-MeP322-Mecp2-pA1-pA2, and scAAV-MeP223-Mecp2-pA1-pA2.

**Table 2: PCR primer sequences for scAAV-MeP426-Mecp2-pA1-pA2, scAAV-MeP322-Mecp2-pA1-pA2, and scAAV-MeP223-Mecp2-pA1-pA2**

| Name | Template sequence | Primer sequence |
|---|---|---|
| MeP426 | MeP549 (SEQ ID NO: 14) | MeP426-F (SEQ ID NO: 28) |
| | | MeP-R (SEQ ID NO: 31) |
| MeP322 | MeP549 (SEQ ID NO: 14) | MeP322-F (SEQ ID NO: 29) |
| | | MeP-R (SEQ ID NO: 31) |
| MeP223 | MeP549 (SEQ ID NO: 14) | MeP223-F (SEQ ID NO: 30) |
| | | MeP-R (SEQ ID NO: 31) |
| Mecp2-pA1-pA2 | Mecp2-pA1-pA2 (SEQ ID NO: 18) | Mecp2-F (SEQ ID NO: 24) |
| | | pA1-pA2-R (SEQ ID NO: 32) |

(5) Construction of scAAV-MeP426-EGFP(c-myc)-pA49:
The scAAV backbone plasmid (PackGene Biotech, EC021) was digested with NheI-HF (NEB, R3131V) and HindIII-HF (NEB, R3104V) to excise a CMV promoter. MeP426 was amplified by PCR, and its template and primers were shown in Table 3. The amplified product was ligated to the linear scAAV backbone via seamless cloning (Clone Smarter, C5891-50). The ligation product was transformed into recombinase-deficient E. coli competent cells stabl3 (TransGen, CD521-01 ) for amplification, thereby constructing scAAV-MeP426-EGFP-WPRE-pA (SV40).

The constructed scAAV-MeP426-EGFP-WPRE-pA (SV40) plasmid was digested with BsrGI-HF (NEB, R3575V) and NotI-HF (NEB, R3189V) to remove a WPRE-pA (SV40) element. c-myc-pA49 was amplified by PCR, and its template and primers were shown in Table 3. The amplified product was ligated to the linear scAAV backbone via seamless cloning (Clone Smarter, C5891-50). The ligation product was transformed into recombinase-deficient E. coli competent cells stabl3 (TransGen, CD521-01) for amplification, thereby constructing scAAV-MeP426-EGFP(c-myc)-pA49.

**Table 3 PCR primer sequences for scAAV-MeP426-EGFP(c-myc)-pA49**

| Name | Template sequence | Primer sequence |
|---|---|---|
| MeP426 | scAAV-MeP426-Mecp2-pA1-pA2 (SEQ ID NO: 42) | MeP426-EGFP-F (SEQ ID NO: 57) |
| | | MeP426-EGFP-R (SEQ ID NO: 58) |
| c-myc-pA49 | scAAV-MeP549-Mecp2-pA49 (SEQ ID NO: 20) | myc-pA49-F (SEQ ID NO: 59) |
| | | myc-pA49-R (SEQ ID NO: 60) |

### 1.3 Construction of scAAV Vectors Comprising miRNA Binding Sites

The detailed construction steps were as follows:
(1) The scAAV-MeP426-Mecp2-pA1-pA2, scAAV-MeP322-Mecp2-pA1-pA2, and scAAV-MeP223-Mecp2-pA1-pA2 plasmids constructed in 1.2 were linearized by digestion with BsaI-HFv2 (NEB, R3733S).

**Table 4: PCR Primer Sequences for miRNA Clusters**

| Name | Primer name | Primer sequence |
|---|---|---|
| 183-483-122 | 183-483-122-F | SEQ ID NO: 34 |
| | 183-483-122-R | SEQ ID NO: 35 |
| 183-483-122-124 | 183-483-122-124-F | SEQ ID NO: 36 |
| | 183-483-122-124-R | SEQ ID NO: 37 |
| 183-483-122-130 | 183-483-122-130-F | SEQ ID NO: 38 |
| | 183-483-122-130-R | SEQ ID NO: 39 |
| 183-483-122-132 | 183-483-122-132-F | SEQ ID NO: 40 |
| | 183-483-122-132-R | SEQ ID NO: 41 |

(2) miRNA cluster sequences 183-483-122, 183-483-122-124, 183-483-122-130, and 183-483-122-132 were synthesized in the form of long primers. The primer sequences were shown in Table 4, and the two primers were used as templates for each other to amplify and produce the required fragments.
(3) The above amplified fragments were ligated to the linearized vectors (scAAV-MeP426-Mecp2-pA1-pA2, scAAV-MeP322-Mecp2-pA1-pA2, and scAAV-MeP223-Mecp2-pA1-pA2) via seamless cloning. The ligation products were transformed into recombinase-deficient E. coli competent cells stabl3 (TransGen, CD521-01) for amplification. The obtained AAV vectors were shown below, and the sequences of the exogenous nucleic acid fragments between their ITR sequences were as follows:
scAAV-MeP426-Mecp2-pA1-pA2 (as shown in SEQ ID NO: 42), scAAV-MeP426-Mecp2-pA1-pA2-183-483-122 (as shown in SEQ ID NO: 43), scAAV-MeP426-Mecp2-pA1-pA2-183-483-122-124 (as shown in SEQ ID NO: 44), scAAV-MeP426-Mecp2-pA1-pA2-183-483-122-130 (as shown in SEQ ID NO: 45), and scAAV-MeP426-Mecp2-pA1-pA2-183-483-122-132 (as shown in SEQ ID NO: 46).
scAAV-MeP322-Mecp2-pA1-pA2 (as shown in SEQ ID NO: 47), scAAV-MeP322-Mecp2-pA1-pA2-183-483-122 (as shown in SEQ ID NO: 48), scAAV-MeP322-Mecp2-pA1-pA2-183-483-122-124 (as shown in SEQ ID NO: 49), scAAV-MeP322-Mecp2-pA1-pA2-183-483-122-130 (as shown in SEQ ID NO: 50), and scAAV-MeP322-Mecp2-pA1-pA2-183-483-122-132 (as shown in SEQ ID NO: 51).
scAAV-MeP223-Mecp2-pA1-pA2 (as shown in SEQ ID NO: 52), scAAV-MeP223-Mecp2-pA1-pA2-183-483-122 (as shown in SEQ ID NO: 53), scAAV-MeP223-Mecp2-pA1-pA2-183-483-122-124 (as shown in SEQ ID NO: 54), scAAV-MeP223-Mecp2-pA1-pA2-183-483-122-130 (as shown in SEQ ID NO: 55), and scAAV-MeP223-Mecp2-pA1-pA2-183-483-122-132 (as shown in SEQ ID NO: 56).

Taking scAAV-MeP426-Mecp2-pA1-pA2-183-483-122-130 as an example, the order of its elements was shown in FIG. 1, and FIG. 16 was a schematic diagram of the miRNA binding sites contained in this vector.

### Example 2: Identification of Expression Efficiency of Expression Vectors

### 2.1 Identification of In Vitro Expression Efficiency of Vector Transfection

Using the vectors constructed in Example 1, Neuro-2a cells (Zhong Qiao Xin Zhou, ZQ0207) were transfected with the transfection reagent PEIpro (Polyplus, 101000026). The C-myc antibody tag (Cell Signaling Technology, 2276S) for detecting the expression of the target gene was used in Western blot experiments to determine the expression level of the Mecp2 protein. Meanwhile, the antibody against an internal reference protein tubulin (Proteinates, 66240-1-Ig) was detected.

The steps were as follows: a primary antibody was incubated with shaking overnight in a 4°C refrigerator. After complete incubation of the primary antibody, a secondary antibody conjugated with horseradish peroxidase-Peroxidase AffiniPure Goat Anti-Mouse IgG (H+L) (Jackson ImmunoResearch, 115-035-003)-was incubated. After complete incubation, color development was performed using ECL chemiluminescent solution (Share-Bio, SB-WB012).

The results were shown in FIG. 2, where different labels in the figure represented transfection with different vectors:
#1 represents scAAV-MeP223-Mecp2-pA1-pA2,
#2 represents scAAV-MeP223-Mecp2-pA1-pA2-183-483-122,
#3 represents scAAV-MeP223-Mecp2-pA1-pA2-183-483-122-124,
#4 represents scAAV-MeP223-Mecp2-pA1-pA2-183-483-122-130,
#5 represents scAAV-MeP223-Mecp2-pA1-pA2-183-483-122-132,
#6 represents scAAV-MeP322-Mecp2-pA1-pA2,
#7 represents scAAV-MeP322-Mecp2-pA1-pA2-183-483-122,
#8 represents scAAV-MeP322-Mecp2-pA1-pA2-183-483-122-124,
#9 represents scAAV-MeP322-Mecp2-pA1-pA2-183-483-122-130,
#10 represents scAAV-MeP322-Mecp2-pA1-pA2-183-483-122-132,
#11 represents scAAV-MeP426-Mecp2-pA1-pA2,
#12 represents scAAV-MeP426-Mecp2-pA1-pA2-183-483-122,
#13 represents scAAV-MeP426-Mecp2-pA1-pA2-183-483-122-124,
#14 represents scAAV-MeP426-Mecp2-pA1-pA2-183-483-122-130,
#15 represents scAAV-MeP426-Mecp2-pA1-pA2-183-483-122-132,
EGFP represents scAAV-MeP426-EGFP(c-myc)-pA49,
#549 represents scAAV-MeP549-Mecp2-pA49, and
Blank represented an untransfected blank group.

The results in FIG. 2 showed that, compared with the untransfected group (blank) and the experimental control group (scAAV-MeP426-EGFP(c-myc)-pA49), all vectors constructed in Example 1 expressed the MeCP2 protein to varying degrees. Among them, compared with scAAV-MeP549-Mecp2-pA49 without an miRNA binding site, all 15 vectors in the present application reduced the expression level of the MeCP2 protein in nerve cells. This indicates that when the vector contained the first miRNA binding site (comprising one or more of a miR-22 binding site, a miR-132 binding site, a miR-124 binding site, a miR-483 binding site, or a miR-130 binding site), it could express the MeCP2 protein in neuron, and the expression level of the MeCP2 protein is not excessively high. In addition, among these 15 vectors, 4 vectors could reduce the expression level of the MeCP2 protein in neuron more significantly (comprising scAAV-MeP223-Mecp2-pA1-pA2-183-483-122-130, scAAV-MeP322-Mecp2-pA1-pA2-183-483-122-130, scAAV-MeP426-Mecp2-pA1-pA2-183-483-122-124, and scAAV-MeP426-Mecp2-pA1-pA2-183-483-122-130), especially the vector scAAV-MeP426-Mecp2-pA1-pA2-183-483-122-130.

### 2.2 Comparison of In Vitro Expression Effects of Vectors

According to the vector construction method in Example 1, scAAV vectors containing control miRNA binding sites were constructed. The construction method was briefly described as follows:
(1) The vector scAAV-MeP426-Mecp2-pA1-pA2 obtained in Example 1 was subjected to circular PCR, and a control miRNA binding site was introduced. The PCR primer sequences for the control miRNA binding site were shown in Table 5 below.

**Table 5: PCR Primer Sequences for Control miRNA**

| Name | Primer name | Primer sequence |
|---|---|---|
| hsa-let-7a-1 | let-7a-1-F | SEQ ID NO: 61 |
| | let-7a-1-R | SEQ ID NO: 62 |
| has-miR-99a-5p | 99a-5p-F | SEQ ID NO: 63 |
| | 99a-5p-R | SEQ ID NO: 64 |
| hsa-miR-100-5p | 100-5p-F | SEQ ID NO: 65 |
| | 100-5p-R | SEQ ID NO: 66 |
| hsa-miR-802 | 802-F | SEQ ID NO: 67 |
| | 802-R | SEQ ID NO: 68 |
| hsa-miR-1200 | 1200-F | SEQ ID NO: 69 |
| | 1200-R | SEQ ID NO: 70 |

(2) Amplification primers for control miRNAs (hsa-let-7a-1, hsa-miR-99a-5p, hsa-miR-100-5p, hsa-miR-802, and hsa-miR-1200) were synthesized in the form of long primers. The primer sequences were shown in Table 5. Circular PCR was performed on the vector scAAV-MeP426-Mecp2-pA1-pA2 to introduce an miRNA binding site.
(3) The above amplified fragments were circularized via seamless cloning. The ligation products were transformed into recombinase-deficient E. coli competent cells stabl3 (TransGen, CD521-01) for amplification. The obtained AAV vectors were shown below, and the sequences of the exogenous nucleic acid fragments between their ITR sequences were as follows:
scAAV-MeP426-Mecp2-pA1-pA2-let-7a-1 (as shown in SEQ ID NO: 71),
scAAV-MeP426-Mecp2-pA1-pA2-99a-5p (as shown in SEQ ID NO: 72),
scAAV-MeP426-Mecp2-pA1-pA2-100-5p (as shown in SEQ ID NO: 73),
scAAV-MeP426-Mecp2-pA1-pA2-802 (as shown in SEQ ID NO: 74), and
scAAV-MeP426-Mecp2-pA1-pA2-1200 (as shown in SEQ ID NO: 75).

After construction, according to the method in Example 2.1, the 5 scAAV vectors comprising the control miRNA binding site obtained above were transfected into Neuro-2a cells. Meanwhile, the vector scAAV-MeP426-Mecp2-pA1-pA2-183-483-122-130 from Example 2.1 was also used in the experiment. Subsequently, the expression level of the MeCP2 protein was detected by Western blot, and the results were shown in FIG. 3. Among them, different labels in the figure represented transfection with different vectors:
Blank represented an untransfected blank group,
PC represents scAAV-MeP426-Mecp2-pA1-pA2,
14 represents scAAV-MeP426-Mecp2-pA1-pA2-183-483-122-130,
Let-7a-1 represents scAAV-MeP426-Mecp2-pA1-pA2-let-7a-1,
802 represents scAAV-MeP426-Mecp2-pA1-pA2-802,
99a-5p represents scAAV-MeP426-Mecp2-pA1-pA2-99a-5p,
100-5p represents scAAV-MeP426-Mecp2-pA1-pA2-100-5p, and
1200 represents scAAV-MeP426-Mecp2-pA1-pA2-1200.

The results in FIG. 3 showed that, compared with the 5 kinds of first miRNA binding sites provided in the present application (the miR-22 binding site, the miR-132 binding site, the miR-124 binding site, the miR-483 binding site, or the miR-130 binding site), other miRNA binding sites (e.g., miR-let-7a-1, miR-802, miR-99a-5p, miR-100-5p, miR-1200, etc.) could not effectively reduce the expression of the target gene.

In addition ,apart from miR-let-7a-1, miR-802, miR-99a-5p, miR-100-5p, and miR-1200, the applicant also found a large number of miRNA binding sites that could not effectively reduce the expression efficiency of the target gene during the screening process. This confirmed that the strategy of reducing target gene expression through miRNA binding sites was affected by multiple factors such as miRNA type, length and type of the target gene, and species type, and thus had unpredictability. Therefore, the 5 kinds of first miRNA binding sites provided in the present application were the most preferred for reducing the expression level of the Mecp2 gene in human brain tissue.

### 2.3 Identification of In Vitro Expression Efficiency of Vector Infection

Using the vectors constructed in Example 1, AAV9 serotype viruses were packaged according to the method disclosed in the literature (Mol Ther. 2013, 21(1):18-30.), which were then used to infect Neuro-2a cells. The expression level of Mecp2 after cell infection with different vectors was determined using the method in 2.1.

The results were shown in FIG. 4, where different labels in the figure represented infection with different vectors:
#6 represents scAAV-MeP322-Mecp2-pA1-pA2,
#7 represents scAAV-MeP322-Mecp2-pA1-pA2-183-483-122,
#8 represents scAAV-MeP322-Mecp2-pA1-pA2-183-483-122-124,
#9 represents scAAV-MeP322-Mecp2-pA1-pA2-183-483-122-130,
#10 represents scAAV-MeP322-Mecp2-pA1-pA2-183-483-122-132,
#11 represents scAAV-MeP426-Mecp2-pA1-pA2,
#12 represents scAAV-MeP426-Mecp2-pA1-pA2-183-483-122,
#13 represents scAAV-MeP426-Mecp2-pA1-pA2-183-483-122-124,
#14 represents scAAV-MeP426-Mecp2-pA1-pA2-183-483-122-130,
#15 represents scAAV-MeP426-Mecp2-pA1-pA2-183-483-122-132,
EGFP represents scAAV-MeP426-EGFP(c-myc)-pA49,
#549 represents scAAV-MeP549-Mecp2-pA49, and
Blank represented an uninfected blank group.

The results in FIG. 4 showed that, compared with the uninfected group (blank) and the experimental control group (scAAV-MeP426-EGFP(c-myc)-pA49), all vectors constructed in Example 1 expressed the MeCP2 protein to varying degrees, , which further verified the results in Example 2.1. In addition, compared with vectors using an MeP322 promoter (corresponding to lanes #7, #8, #9, #10 in FIG. 4), a vector using the MeP426 promoter could further reduce the expression level of the MeCP2 protein in neuron (corresponding to lanes #12, #13, #14, #15 in FIG. 4). The above results indicated that on the basis of selecting specific first miRNA binding sites (the miR-22 binding site, the miR-132 binding site, the miR-124 binding site, the miR-483 binding site, or the miR-130 binding site), using the MeP426 promoter in the gene expression cassette could further reduce the expression level of the MeCP2 protein in nerve cells, achieving a better effect.

### 2.4 Identification of In Vivo Expression Efficiency of Vector Infection

Using scAAV-pMeP549-Mecp2-pA49 constructed in Example 1, three serotypes (AAV1, AAV8, AAV9) were packaged according to the method disclosed in the literature (Mol Ther. 2013, 21(1):18-30.). Intraventricular injection was performed on the P1 ventricle of wild-type C57 mice. Each mouse received bilateral intraventricular injections (left and right ventricles) at a dose of 5×10¹⁰ vg, and 3 animals were injected for each serotype. Four weeks after injection, the brain, spinal cord, and liver were collected, and the expression level of MeCP2 was determined by Western blot using the method in 2.1.

The results were shown in FIG. 5. The results indicated that the three serotypes of scAAV-pMeP549-Mecp2-pA49 could express the MeCP2 protein in the mouse brain to varying degrees; therefore, they might have a good therapeutic effect on Rett syndrome (Brain. 2021, 144(10): 3005-3019).

### Example 3: Therapeutic Effects of Different Expression Vectors on Rett Model Mice

### 3.1 Construction Method of Rett Model Mice

B6.129P2(C)-Mecp2tm1.1Bird/J transgenic mice (Jax.003890) were purchased from Jackson laboratory. Hemi mice with the genotype Mecp2-/y, which served as the experimental Rett model mice, were obtained by breeding Mecp2+/- female mice with wild-type C57BL/6J mice.

### 3.2 Vector Construction Method

The vectors constructed in Example 1 were packaged into two serotypes, AAV1 and AAV9, according to the method disclosed in the literature (Mol Ther. 2013, 21(1):18-30.), where:
8241 represented the vector scAAV9-MeP549-Mecp2-pA49 with a serotype of AAV9,
8243 represented the vector scAAV9-MeP322-Mecp2-pA1-pA2-183-483-122-130 with a serotype of AAV9,
8244 represented the vector scAAV9-MeP426-Mecp2-pAl-pA2-183-483-122-130 with a serotype of AAV9,
8245 represented the vector scAAV9-MeP426-Mecp2-pA1-pA2-183-483-122-132 with a serotype of AAV9, and
8246 represented the vector scAAV1-MeP426-Mecp2-pA1-pA2-183-483-122-132 with a serotype of AAV1.

### 3.3 Survival Period Detection

After the birth of Rett model mice, Mecp2-/y neonatal mice were selected for the experiment through genotype identification. Within 24 hours of birth, the offspring mice were lateral intraventricularly injected with a vehicle (N=5), or 8244 (N=7), 8241 (N=6), 8243 (N=6), 8245 (N=6), or 8246 (N=7) at a dose of 5.0×10¹⁰ vg per mouse. In addition, neonatal mice at 7 days after birth (PND7) were lateral intraventricularly administered 8245 (N=6) at a dose of 5.0×10¹⁰ vg per mouse. The administration volume per mouse was 2.5 µL per lateral ventricle.

The survival curve results were shown in FIG. 6. The median survival time of the Rett model mice in the untreated vehicle group was 66 days. At a dose of 5×10¹⁰ vg per mouse, the median survival time of model mice in the 8244 treatment group was 175 days; in the 8241 treatment group, it was 78.5 days; in the 8243 treatment group, it was 166.5 days; in the 8245 treatment group, it was 134.5 days; and in the 8246 treatment group, it was 108 days. These results indicated that all vectors provided in the present application could effectively prolong the survival period of the Rett model mice, where 8244 could better prolong the median survival time of the model mice. Furthermore, the median survival time of PND7 model mice treated with 8245 (administered at 7 days after birth) was 108 days, while that of PND1 model mice was 134.5 days. This suggested that administration within 24 hours of mouse birth yielded a better improvement in survival period.

### 3.4 Behavioral Detection

After the birth of Rett model mice, Mecp2-/y neonatal mice were selected for the experiment through genotype identification. Within 24 hours of birth, the offspring mice were lateral intraventricularly injected with a vehicle (N=5), or 8244 (N=6), 8241 (N=4), 8243 (N=6), 8245 (N=5), or 8246 (N=6) at a dose of 5.0×10¹⁰ vg per mouse. In addition, neonatal mice at 7 days after birth (PND7) were lateral intraventricularly administered 8245 (N=6) at a dose of 5.0×10¹⁰ vg per mouse. After 50 days of administration, gait analysis was performed on the administered model mice and wild-type mice.

A gait tester (Catwalk, Noldus, CatWalk XT 10.1) was used to evaluate the gait and movement of the mice. The specific testing method was as follows:
(1) Experimental animals were transferred to the experimental room in advance to adapt to the dark environment;
(2) The length of the instrument runway was set to 3-6 times the body length of the animals ,ensuring the animal takes 4-6 steps. The track position was adjusted to be parallel, and a scale was set under these parameters;
(3) Animals were placed on the starting side of the runway. The system automatically detected when the animals entered the monitoring range and started recording. After each successful run, an experimental record was generated. Testing for each animal was stopped once three successful experimental records were obtained, and then the next animal was tested;
(4) Any stains on the runway were cleaned in a timely manner to ensure the quality of the collected data; and
(5) Gait data of all animals were statistically analyzed.

The results of the gait analysis were shown in FIGS. 7-9. The results indicated that all vectors provided in the present application could effectively improve the regularity index (RI) of Rett model mice, and also improve the swing speed and stride length to varying degrees (where RF represented the right forelimb, RH represented the right hindlimb, LF represented the left forelimb, and LH represented the left hindlimb). The above results demonstrated that all vectors provided in the present application could effectively alleviate symptoms related to Rett syndrome.

In addition, among all vectors provided in the present application, the gait regularity index of all treated mice in the 8244 group (N=6) recovered to the normal level, while there were still mice with low gait regularity index in other vector groups. Meanwhile, the swing speed and stride length of mice in the 8244 group during movement reached the same level as those of wild-type mice, indicating that 8244 had the best therapeutic effect.

### Example 4: Therapeutic Effects of Different Doses of Expression Vectors on Rett Model Mice

The construction methods of Rett model mice and the expression vector 8244 were the same as those in Example 3.

### 4.1 Survival Period Detection

After the birth of the transgenic mice, Rett model neonatal mice were selected for the experiment through genotype identification. 8244 was administered to the mice at different doses: 1.75×10¹⁰ vg per mouse (Dose 5), 4.38×10¹⁰ vg per mouse (Dose 4), 8.75×10¹⁰ vg per mouse (Dose 3), 1.75×10¹¹ vg per mouse (Dose 2), and 3.5×10¹¹ vg per mouse (Dose 1).

The survival curve results were shown in FIG. 10. The results showed that a median survival time of Rett model mice in the vehicle group untreated with the 8244 vector was 54 days. For the dose groups, a median survival time of mice in the 1.75×10¹⁰ vg per mouse was 63 days, a median survival time of mice in the 4.38×10¹⁰ vg per mouse was 89.5 days (with the longest survival period exceeding 185 days), a median survival time of mice in the 8.75×10¹⁰ vg per mouse group was 135.5 days; and a median survival time of mice in the 1.75×10¹¹ vg per mouse was 100.5 days. The above results confirmed that within the dose window of 4.38×10¹⁰ vg per mouse to 1.75×10¹¹ vg per mouse, the survival period of Rett model mice was significantly prolonged as the dose increases.

However, the results also showed that at a high dose of 3.5×10¹¹ vg per mouse, the median survival time of the model mice was only 30.5 days, and the survival period was not prolonged. This confirmed that an excessively high injection dose led to the expression of excessive MeCP2 protein, which in turn produced toxicity and affected the therapeutic effect on the mice.

### 4.2 Behavioral Detection

(1) The detection method of the rotarod test was as follows:
   A rotarod tester (Harvard Apparatus, Panlab Rotarod, LE8205, USA) was used for the rotarod test. Animals underwent pre-training on the rotarod at P69 to adapt to the equipment and environment, and the rotarod test was conducted at P70-P71. The rotarod tester rotated at a constant speed during the training phase and at a uniformly accelerated speed during the test phase. The time the mice stayed on the rotarod tester was recorded.

Training phase: P69 animals were placed on the rotarod tester, and they were required to maintain balance and stability on the rotarod tester rotating at a constant speed of 4 rpm for 5 minutes. Considering that the experimental animals in this project were a movement dysfunction model, if the animals could not persist for 5 minutes, training was continued as much as possible until no further progress could be made.

Test phase: from P70 to P71, the animals were subjected to 3 uniformly accelerated rotarod tests (accelerating from 4 rpm to 40 rpm within 5 minutes, with an angular acceleration of 0.72°/s2) on the rotarod tester. The time from the start of the experiment until the animal fell off due to inability to maintain balance was recorded. For animals that did not fall off within the 5-minute test period, the drop time was recorded as the maximum test duration. The interval between each test was set to 5 minutes. Before the start of the next test, all equipment was cleaned with 75% alcohol and thoroughly deodorized.

(2) The detection results of the rotarod test were shown in FIG. 11. After 70-71 days of administration, the surviving mice were subjected to the rotarod test 4 times a day for 2 consecutive days. The time each mouse persisted on the rotarod in each of the 8 tests was counted. The results showed that in the 4 tests on the second day, the time the mice in the vehicle group (N=3) persisted on the rotarod was significantly different from that of the wild-type mice (N=10), indicating that the motor ability and muscle endurance of the model mice were impaired. For the dose groups,1.75×10¹⁰ vg per mouse (N=3), 4.38×10¹⁰ vg per mouse (N=5), and 8.75×10¹⁰ vg per mouse (N=8), there was no significant difference in the time the mice fell off the rotarod compared with the wild-type mice (N=10) in the 8 tests. This indicated that after treating Rett model mice with the 8244 vector at the three doses of 1.75×10¹⁰ vg per mouse (N=3), 4.38×10¹⁰ vg per mouse (N=5), and 8.75×10¹⁰ vg per mouse (N=8), the motor coordination ability and muscle endurance of the model mice could be effectively restored.

However, in the high-dose groups of 1.75×10¹¹ vg per mouse (N=7) and 3.5×10¹¹ vg per mouse (N=1), the time the mice persisted on the rotarod was shorter than that of the mice in the vehicle group (N=3). This indicated that an excessively high dose of the virus led to the expression of excessive MeCP2 protein, thereby affecting the therapeutic effect on the mice.

### Example 5: Effects of Expression Vectors on Protein Expression and Liver Tissue Damage in Mice

The construction methods of Rett model mice and the expression vector were the same as those in Example 3. Single intraventricular injections of the expression vectors 8244, 8241, 8243, 8245, and 8246 were administered to 0-1 day-old Rett model mice. 28 days later, the brain and spinal cord tissues were collected, and the brain, spinal cord, and liver were collected. Half of the tissues were immersed and fixed in 4% paraformaldehyde for tissue damage detection; and the other half were quickly frozen in liquid nitrogen and stored in a -80°C refrigerator for in vivo protein expression detection.

### 5.1 Detection of Protein Expression in Mice

The tissues frozen in liquid nitrogen were homogenized to extract proteins. Western blot as described in Example 2 was used to detect the expression level of the MeCP2 protein, and the results were shown in FIG. 12. The results showed that compared with Rett model mice treated with the vehicle, all vectors provided in the present application could express a certain level of the MeCP2 protein in the brain and spinal cord of the central nervous system. Among them, the level of MeCP2 expressed by the expression vector 8244 was relatively close to that of wild-type mice, suggesting that the expression vector 8244 is a more preferred treatment option.

### 5.2 Detection of Liver Tissue Damage in Mice

Pathological section staining was used to detect liver tissue damage, and the method was as follows:
(1) Tissue sampling and fixation: Tissue blocks (generally no more than 0.5 cm in thickness) were taken from the fresh cadavers of humans or animals and immersed in a pre-prepared fixation fluid (such as 10% formalin, Bouin's fixation fluid, etc.) to denature and coagulate the proteins in the tissues and cells. This prevented autolysis of the cells after death or decomposition by bacteria, thereby preserving the original morphological structure of the cells.
(2) Dehydration and clearing:
   Generally, alcohol with low to high concentrations was used as a dehydrating agent to gradually remove water from the tissue blocks. The tissue blocks were then placed in a transparent agent xylene that was soluble in both alcohol and paraffin to replace the alcohol in the tissue blocks with xylene. This step was necessary before wax immersion and embedding.
(3) Wax immersion and embedding:
   The cleared tissue blocks were placed in melted paraffin and incubated in a wax-melting oven. After the paraffin was completely immersed in the tissue blocks, embedding was performed: a container (such as a folded small paper box) was prepared first, melted paraffin was poured into it, and the tissue blocks soaked in paraffin were quickly clamped and placed inside. After cooling and solidification into a block, the embedding process was completed. The embedded tissue blocks became hard enough to be cut into thin sections on a microtome.
(4) Cutting into sections and pasting sections:
   The embedded wax block was fixed on a microtome and cut into thin sections, generally 5-8 µm thick. The cut thin sections were often wrinkled, so they were placed in heated water to flatten them, then pasted on glass slides, and dried in a 45°C constant-temperature oven.
(5) Dewaxing and staining: HE staining was commonly used to increase the color difference between different parts of the tissue and cell structure for easier observation. Hematoxylin (H) was a basic dye that could stain cell nuclei and ribosomes in cells blue-purple; structures stained by a basic dye were basophilic. Eosin (E) was an acidic dye that could stain cytoplasm red or light red; structures stained by an acidic dye were eosinophilic. Before staining, the paraffin in the sections must be removed with xylene, followed by treatment with alcohol with high to low concentrations, and finally immersion in distilled water for staining.

HE staining process: the sections after immersion in distilled water were placed in hematoxylin aqueous solution for staining for several minutes. Color separation was performed in acid water and ammonia water, each for several seconds. After rinsing with running water for 1 hour, the sections were immersed in distilled water for a short time. They were then soaked in 70% alcohol and 90% alcohol for dehydration for 10 minutes each. They were then soaked in alcoholic eosin staining solution for 2-3 minutes.
(6) Dehydration and clearing: the stained sections were dehydrated with absolute alcohol and then cleared with xylene.
(7) Sealing: Canadian gum was dropped onto the cleared sections, and a coverslip was placed on top for sealing. After the gum dried slightly, a label was attached, and the section specimen was ready for use.

The results of the pathological sections were shown in FIGS. 13-15. The results indicated that, compared with the control group, none of the vectors provided in the present application caused significant liver tissue damage. This confirmed that adding a third miRNA binding site (e.g., a miR-122 binding site) to the vector could effectively reduce liver toxicity induced by AAV administration. In addition, none of the vectors provided in the present application caused obvious dorsal root ganglion lesions (results not shown), which confirmed that adding a second miRNA binding site (e.g., a miR-183 binding site) to the vector could effectively reduce dorsal root ganglion toxicity induced by AAV administration.

In addition, the results in FIGS. 13-15 also showed that only a small number of mild pathological changes were observed in the liver tissues of animals in each group, such as: a small amount of hepatic vacuolar degeneration around the hepatic veins in animal 2 of group 8241; vacuolar degeneration of liver cells around the portal area and an increase in kupffer cells (macrophages/tissue cells) in the hepatic sinusoids in animal 2 of group 8243; mild proliferation of bile duct epithelial cells in the portal area of animal 1 of group 8245; and liver cell ballooning and vacuolar degeneration of animal 2 of group 8245. Meanwhile, compared with the 8241, 8243, and 8245 groups, no mild pathological changes were found in the liver sections of the 3 animals in the 8244 group. This confirmed that 8244 can further reduce the impact of the AAV vector on the liver and was a more preferred treatment option.

It should be noted that the above are only preferred embodiments of the present application and are not intended to limit the present application. For those skilled in the art, various modifications and changes may be made to the present application. Although specific embodiments have been described, alternatives, modifications, changes, improvements, and substantial equivalents of the above embodiments may exist or be unforeseeable at present for the applicant or other skilled in the art. Therefore, the appended claims submitted and any claims that may be modified are intended to cover all such alternatives, modifications, changes, improvements, and substantial equivalents. Importantly, with the evolution of technology, many elements described herein may be replaced by equivalent elements that appear after the present application.

## Claims

1. A gene expression cassette comprising a polynucleotide sequence encoding a human MeCP2 protein, wherein the gene expression cassette comprises at least one first miRNA binding site, wherein the first miRNA binding site includes one or more of a miR-22 binding site, a miR-132 binding site, a miR-124 binding site, a miR-483 binding site, or a miR-130 binding site.

2. The gene expression cassette according to claim 1, wherein the first miRNA binding site is located in a 3' transcription termination sequence of the gene expression cassette, wherein the 3' transcription termination sequence includes a distal termination sequence and/or a proximal termination sequence.

3. The gene expression cassette according to claim 2, wherein the proximal termination sequence comprises a miR-22 binding site.

4. The gene expression cassette according to claim 2, wherein the distal termination sequence comprises at least one of a miR-132 binding site, a miR-124 binding site, a miR-483 binding site, or a miR-130 binding site.

5. The gene expression cassette according to claim 1, wherein the first miRNA binding site includes at least one of the following groups:
(1) a miR-22 binding site, a miR-132 binding site, a miR-124 binding site, and a miR-483 binding site; and/or
(2) a miR-22 binding site, a miR-132 binding site, a miR-124 binding site, a miR-483 binding site, and a miR-130 binding site.

6. The gene expression cassette according to claim 5, wherein the first miRNA binding site includes at least one of the following groups:
(1) 1 miR-22 binding site, 1 miR-132 binding site, 1 miR-124 binding site, and 1 miR-483 binding site;
(2) 1 miR-22 binding site, 1 miR-132 binding site, 2 miR-124 binding sites, and 1 miR-483 binding site;
(3) 1 miR-22 binding site, 1 miR-132 binding site, 1 miR-124 binding site, 1 miR-483 binding site, and 1 miR-130 binding site; and/or
(4) 1 miR-22 binding site, 2 miR-132 binding sites, 1 miR-124 binding site, and 1 miR-483 binding site.

7. The gene expression cassette according to any one of claims 1-6, wherein the first miRNA binding site can reduce the expression efficiency of the polynucleotide sequence encoding the human MeCP2 protein in human brain tissue.

8. The gene expression cassette according to any one of claims 1-6, wherein at least one thymine nucleotide in the nucleotide sequence of the miR-483 and/or miR-130 binding site is replaced with an adenine nucleotide.

9. The gene expression cassette according to claim 8, wherein:
the nucleotide sequence of the miR-22 binding site includes GGCAGCT;
the miR-132 binding site includes the nucleotide sequence shown in SEQ ID NO: 6;
the miR-124 binding site includes the nucleotide sequence shown in SEQ ID NO: 7;
the miR-483 binding site includes the nucleotide sequence shown in SEQ ID NO: 4; and/or
the miR-130 binding site includes the nucleotide sequence shown in SEQ ID NO: 5.

10. The gene expression cassette according to claim 1, wherein the gene expression cassette further comprises a second miRNA binding site and/or a third miRNA binding site, wherein the second miRNA binding site and/or the third miRNA binding site includes one or more of a miR-183 binding site, a miR-182 binding site, a miR-96 binding site, a miR23b binding site, a miR-145 binding site, a miR-148a binding site, a miR-22 binding site, a miR-122 binding site, a miR-143 binding site, a miR-21 binding site, or a miR-192 binding site.

11. The gene expression cassette according to claim 10, wherein the second miRNA binding site includes a miR-183 binding site, wherein the third miRNA binding site includes a miR-122 binding site.

12. The gene expression cassette according to claim 11, wherein the gene expression cassette includes at least one of the following groups:
(1) a miR-22 binding site, a miR-132 binding site, a miR-124 binding site, a miR-483 binding site, a miR-183 binding site, and a miR-122 binding site; and/or
(2) a miR-22 binding site, a miR-132 binding site, a miR-124 binding site, a miR-483 binding site, a miR-130 binding site, a miR-183 binding site, and a miR-122 binding site.

13. The gene expression cassette according to claim 11, wherein the gene expression cassette includes at least one of the following groups:
(1) 1 miR-22 binding site, 1 miR-132 binding site, 1 miR-124 binding site, 1 miR-483 binding site, 1 miR-183 binding site, and 1 miR-122 binding site;
(2) 1 miR-22 binding site, 1 miR-132 binding site, 2 miR-124 binding sites, 1 miR-483 binding site, 1 miR-183 binding site, and 1 miR-122 binding site;
(3) 1 miR-22 binding site, 1 miR-132 binding site, 1 miR-124 binding site, 1 miR-483 binding site, 1 miR-130 binding site, 1 miR-183 binding site, and 1 miR-122 binding site; and/or
(4) 1 miR-22 binding site, 2 miR-132 binding sites, 1 miR-124 binding site, 1 miR-483 binding site, 1 miR-183 binding site, and 1 miR-122 binding site.

14. The gene expression cassette according to any one of claims 10-13, wherein the second miRNA binding site can reduce the expression efficiency of the polynucleotide sequence encoding the human MeCP2 protein in human dorsal root ganglion, wherein the third miRNA binding site can reduce the expression efficiency of the polynucleotide sequence encoding the human MeCP2 protein in human liver tissue.

15. The gene expression cassette according to any one of claims 10-13, wherein
the miR-183 binding site includes the nucleotide sequence shown in SEQ ID NO: 9; and/or
the miR-122 binding site includes the nucleotide sequence shown in SEQ ID NO: 10.

16. The gene expression cassette according to any one of claims 1-15, wherein the first miRNA binding site, the second miRNA binding site, and/or the third miRNA binding site are operably linked via a spacer sequence, wherein the spacer sequence comprises the nucleotide sequence shown in any one of SEQ ID NOs: 11-13.

17. The gene expression cassette according to any one of claims 1-16, wherein the gene expression cassette further includes a promoter, wherein the promoter comprises at least one of a MeCP2 core promoter, a MeCP2 silencer element, or a CNS regulatory element.

18. The gene expression cassette according to claim 17, wherein the promoter includes MeP549, MeP426, MeP322, or MeP223.

19. The gene expression cassette according to claim 18, wherein:
the MeP549 promoter includes the nucleotide sequence shown in SEQ ID NO: 14;
the MeP426 promoter includes the nucleotide sequence shown in SEQ ID NO: 15;
the MeP322 promoter includes the nucleotide sequence shown in SEQ ID NO: 16; and/or
the MeP223 promoter comprises the nucleotide sequence shown in SEQ ID NO: 17.

20. The gene expression cassette according to any one of claims 1-19, wherein a length of the gene expression cassette does not exceed about 2.2 kb.

21. A recombinant vector, wherein the recombinant vector comprises the gene expression cassette according to any one of claims 1-20.

22. The recombinant vector according to claim 21, wherein the recombinant vector includes a recombinant plasmid expression vector or a recombinant viral vector.

23. The recombinant vector according to claim 22, wherein the recombinant viral vector includes a recombinant adenoviral vector, a recombinant adeno-associated viral vector, a recombinant retroviral vector, a recombinant herpes simplex viral vector, or a recombinant vaccinia viral vector.

24. The recombinant vector according to claim 23, wherein the recombinant viral vector is a recombinant adeno-associated viral vector.

25. The recombinant vector according to claim 24, wherein the serotype of the recombinant adeno-associated viral vector includes AAV 1, AAV2, AAV3, AAV4, AAV5, AAV6, AAV7, AAV8, AAV9, AAVrh10, AAV11, AAV12, AAV13, AAV-PHP.B, AAV-PHP.EB, AAVhu37, AAVrh74, or AAVhu68.

26. The recombinant vector according to claim 25, wherein the serotype of the recombinant adeno-associated viral vector includes AAV1, AAV8, or AAV9.

27. A pharmaceutical composition, wherein the pharmaceutical composition includes the gene expression cassette according to any one of claims 1-20 and the recombinant vector according to any one of claims 21-26.

28. The pharmaceutical composition according to claim 27, wherein the form of the pharmaceutical composition includes an intraventricular injection drug, an intravenous injection drug, or an intrathecal injection drug.

29. Use of the gene expression cassette according to any one of claims 1-20, the recombinant vector according to any one of claims 21-26, or the pharmaceutical composition according to any one of claims 27-28 for expressing a MeCP2 protein in cells.

30. The use according to claim 29, wherein the cells are animal cells.

31. The use according to claim 30, wherein the animal cells include Neuro-2a cells.

32. Use of the gene expression cassette according to any one of claims 1-20, the recombinant vector according to any one of claims 21-26, or the pharmaceutical composition according to any one of claims 27-28 in the preparation of a drug for preventing, alleviating, or treating a disease.

33. The use according to claim 32, wherein the disease is a MeCP2-related disease.

34. The use according to claim 33, wherein the MeCP2-related disease includes: a disease related to insufficient MeCP2 protein expression, a disease related to MeCP2 protein deficiency, a disease related to MeCP2 gene deletion, or a disease related to MeCP2 gene mutation.

35. The use according to claim 34, wherein the MeCP2-related disease is Rett syndrome.

36. The use according to claim 32, wherein the drug includes an intraventricular injection drug, an intravenous injection drug, or an intrathecal injection drug.

37. A method for preventing, alleviating, or treating a disease, including administering the gene expression cassette according to any one of claims 1-20, the recombinant vector according to any one of claims 21-26, or the pharmaceutical composition according to any one of claims 27-28 to a subject in need thereof.

38. The use according to claim 37, wherein the disease is a MeCP2-related disease.

39. The use according to claim 38, wherein the MeCP2-related disease includes: a disease related to insufficient MeCP2 protein expression, a disease related to MeCP2 protein deficiency, a disease related to MeCP2 gene deletion, or a disease related to MeCP2 gene mutation.

40. The use according to claim 39, wherein the MeCP2-related disease is Rett syndrome.
